# FASCICULE DE BREVET EUROPEEN

(11) **EP 3 473 287 B1**
(45) Date de publication et mention de la délivrance du brevet: **28.12.2022**
(21) Numéro de dépôt: 18199807.1
(22) Date de dépôt: 11.10.2018
(51) Int. Cl.: A22B 3/00, A61D 7/04

(54) **PROCEDES D'ANESTHESIE ANIMALE METTANT EN OEUVRE UN RECYCLAGE DES GAZ**
ANÄSTHESIEVERFAHREN FÜR TIERE, BEI DEM EIN GASRECYCLING VERWENDET WIRD
METHODS OF ANIMAL ANAESTHESIA USING GAS RECIRCULATION

(30) Priorité: 17.10.2017 FR 1759716
(43) Date de publication de la demande: 24.04.2019
(73) Titulaire: L'AIR LIQUIDE, SOCIETE ANONYME POUR L'ETUDE ET L'EXPLOITATION DES PROCEDES GEORGES CLAUDE, 75007 Paris (FR); Air Liquide France Industrie, 75007 Paris (FR)
(72) Inventeur: COUSIN, Franck, 75007 PARIS (FR); TAYLOR, Bob, BE-4020 LIEGE (BE); CHARVE, Etienne, 75007 PARIS (FR)
(74) Mandataire: Air Liquide

(56) Documents cités:
- WO-A1-2005/058049
- WO-A1-2008/128027
- WO-A1-2016/193368
- WO-A2-2004/098297
- FR-A1- 2 914 864

## Description

La présente invention concerne le domaine de l'anesthésie animale, et notamment l'anesthésie de mammifères ou gallinacés avant abattage. L'anesthésie permet d'avoir un animal inconscient lors de l'abattage.

La question du bienêtre animal et de celui du personnel est au cœur des préoccupations des instituts de contrôle, tout comme l'est le coût de la méthode d'anesthésie pour l'abattoir.

On sait que dans cette industrie, on trouve essentiellement 3 méthodes d'anesthésie : l'anesthésie électrique, l'étourdissement par choc mécanique et l'anesthésie par l'apport d'un gaz, le plus souvent du CO2 sous sa forme gazeuse.

Les documents FR-2 914 864, WO2004/098297, WO2016/193368 et WO2008/128027, illustrent l'état de la technique de l'anesthésie des animaux avant abattage.

L'anesthésie par apport de CO₂ est couramment pratiquée dans les conditions suivantes :
- le traitement des espèces, en particuliers de gallinacés, est réalisé en mode « batch » (« lot ») ;
- les cages d'animaux vivants arrivent sur le lieu d'abattage dans des boites;
- chaque boite (contenant donc un arrangement de cages) est sortie du camion, véhiculée au sein du lieu d'abattage, entrée dans un sas (ou cellule) de traitement à l'aide du gaz d'anesthésie, sortie du sas une fois le traitement effectué, dirigée vers la chaine d'abattage des animaux (les animaux sont positionné tête en bas sur des crochets défilants etc...) etc.... tout ceci par des moyens de manutention automatique.

Les principaux avantages du CO₂ peuvent être résumés ainsi :
- une anesthésie groupée diminuant le stress animal ;
- la diminution des pétéchies ;
- l'absence d'intervention physique du personnel sur l'animal pour lui faire perdre connaissance.
- une manipulation des animaux dans un état où ils sont inconscients.

Les principaux inconvénients du CO₂ peuvent être résumés ainsi :
- la perte de CO₂ par l'ouverture des portes des SAS ;
- des demandes continues d'optimisation du temps d'inconfort de l'animal ;
- des cadences inférieures à celles des autres méthodes d'anesthésie connues ;
- certes il existe des solutions de recyclage du CO₂ par filtration et concentration, mais qui génèrent alors des coûts d'investissement et des coûts d'énergie électrique importants.

On propose alors dans le cadre de la présente invention une méthode d'anesthésie mettant en œuvre un gaz ou mélange gazeux, qui va être recyclé, permettant d'optimiser la consommation du gaz tout en diminuant le stress animal. Pour cela on propose de recycler le gaz par des moyens de stockages provisoires (que l'on peut appeler capacités), ces moyens de stockages étant, comme on va le voir, à volume variable.

En effet, pour le bien être animal et la qualité finale des produits, le cycle d'anesthésie comporte généralement plusieurs étapes : par exemple le chargement des animaux, puis la montée progressive de la teneur pour l'étourdissement , suivie d'une teneur plus élevée pour atteindre des seuils létaux, et enfin une stabilisation à ces seuils durant une durée prédéterminée (notamment selon l'espèce traitée), avant déchargement pour aller vers l'abattage.

Chacune de ces étapes mettant en œuvre des taux de gaz différents, et par exemple des taux de CO₂ différents, il est avantageux de stocker le gaz recyclé dans des réserves spécifiques.

Pour illustrer ceci, on peut par exemple disposer des stockages suivants :
- une réserve de gaz composée d'air ambiant ou bien de composition proche de l'air ambiant ;
- une réserve de gaz comportant environ 20% de CO₂ ;
- une réserve de gaz comportant environ 40% de CO₂ ;
- une réserve de gaz comportant environ 60% de CO₂ ;
le reste de la composition étant par exemple de l'air.

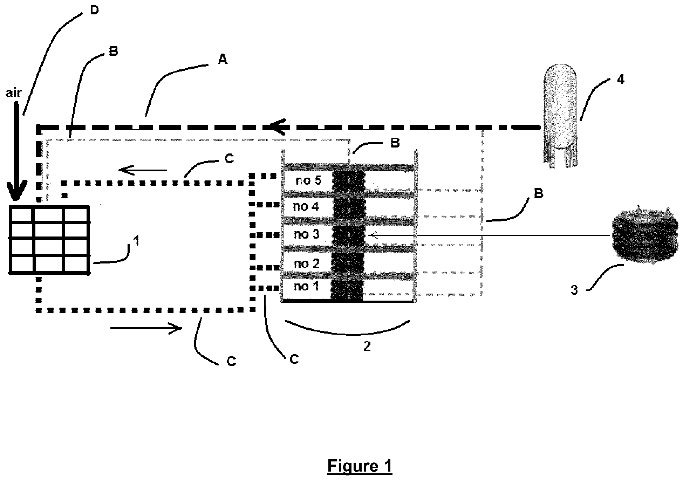

La figure 1 annexée illustre un des modes préférés de mise en oeuvre de l'invention où l'on reconnaît les éléments suivants :
- la référence 1 désigne un sas ou cellule dans lequel sont positionnées les cages contenant les animaux à anesthésier ;
- la référence 2 désigne un ensemble constitué ici (mais ce n'est qu'un exemple) de 5 compartiments (ou « capacités») de stockage de gaz, de capacité variable (on peut aussi appeler ces capacités des « accordéons » ou des « soufflets ») ;
- la vue de détail 3 sur le coté droit de la figure détaille le système qui permet de modifier le volume d'une capacité (dans ce cas illustré ici : un boudin (vérin) pneumatique);
- la référence 4 désigne un stockage de gaz, par exemple du CO₂ liquide ;
- et afin de mieux visualiser les différents mouvements de gaz intervenant dans ce mode de réalisation de l'invention on a utilisé les symboles suivants :
   - à l'extrême gauche de la figure la grosse flèche noire désigne une arrivée d'air « seul » ou « pur » dans le sas 1 (circuit D);
   - la lettre A (trait tireté épais) désigne une arrivée (ou complément) de CO₂ pur dans le sas 1 ;
   - la lettre B (trait tireté fin) désigne la pressurisation au CO₂ pur des boudins, et la dépressurisation des boudins vers le sas ;
   - la lettre C (trait pointillé) désigne le circuit de recyclage du CO₂ entre le sas et l'ensemble 2 des capacités.

Décrivons alors ci-dessous un exemple de mise en œuvre de l'installation de la figure 1 afin de mieux comprendre l'apport de l'invention, en gardant à l'esprit bien entendu le fait que selon les espèces animales traitées, ou encore leur poids, leur âge etc...les temps et les niveaux d'exposition aux gaz devront être adaptés. On illustre ci-dessous un exemple mettant en œuvre du CO₂.

Lors de la mise en service i.e d'une première utilisation, le CO₂ est injecté progressivement dans le sas 1 (circuit A).

Lors de cette 1ere utilisation on réalise l'anesthésie à partir d'un gaz non recyclé («pur »), donc pas à partir de gaz qui aurait été stocké dans l'ensemble 2, mais bien entendu ce gaz injecté pour la 1ere utilisation ne sera pas perdu, il va, comme on va le voir, être stocké dans les capacités 2 pour être utilisé dans le cycle suivant etc....

A ce stade-ci, le sas comprend donc une teneur élevée en CO₂, l'objectif est d'aspirer au mieux cette concentration élevée. Pour segmenter les différentes teneurs de gaz souhaitées, on utilise plusieurs compartiments de stockage de gaz (dans l'exemple donné ici, de No 1 à No 5).

Par exemple, le compartiment N°1 aspire en premier (pour aspirer, on injecte du CO₂ dans le boudin 3 correspondant (circuit B)) l'accordéon se lève et génère une dépression qui aspire du gaz contenu dans le sas (circuit C).

Et on comprend mieux ici le caractère extrêmement avantageux de la proposition technique de l'invention, d'une capacité à volume variable, permettant de créer ainsi la dépression et l'aspiration du gaz contenu dans le sas.

Un analyseur de gaz peut permettre de façon avantageuse de qualifier l'aspiration. A titre illustratif, à 50% de CO₂ dans le compartiment N° 1 on bascule sur le compartiment N°2 selon la démarche décrite ci-dessus.

Un compartiment est surdimensionné en volume pour ne pas perturber le procédé et sous remplir des compartiments en basculant sur le compartiment suivant sans avoir atteint la valeur cible.

Un compartiment suffisamment rempli est obturé par une vanne étanche.

Quand on bascule sur le compartiment N°2, on souhaite par exemple remplir celui-ci par un gaz moins élevé en teneur que le N°1. Pour cela on injecte dans le sas de l'air ambiant, ou bien on ouvre une vanne qui va permettre d'aspirer de l'air frais.

Pour lever le boudin N°2 à nouveau on injecte du CO₂ dans ce boudin.

De façon préférée, l'injection d'air sera réalisée à l'opposée du point d'aspiration. Dans le cas d'un gaz avec une densité élevée comme le CO₂, on préfère une aspiration dans le bas du sas et une injection d'air dans le haut du sas.

Le compartiment 2 rempli, on passe sur le 3 puis le 4 et enfin le 5.

Une fois le traitement réalisé, et donc suite à l'ouverture du sas pour sortir les animaux et introduire un nouveau lot, le sas 1 est donc composé d'une teneur pauvre en gaz anesthésiant (en fait proche de l'air ambiant). On préfère alors injecter du gaz dans le sas de façon progressive.

Il est préférable alors de commencer par le compartiment le moins concentré en gaz anesthésiant (ici dans l'exemple décrit ci-dessus le n°5).

Le processus d'anesthésie étant basé sur un couple temps / teneur en gaz, un analyseur couplé à une régulation de temps pourra avantageusement piloter l'ouverture des différents compartiments pour respecter les valeurs de gaz nécessaires (ouverture des boudins N°5 à N°1).

Pour libérer le gaz d'un compartiment, il convient d'ouvrir la vanne correspondante mais aussi purger le boudin, sachant que le gaz utilisé pour actionner le vérin est justement le gaz anesthésiant il sera avantageusement libéré dans le sas pour être valorisé.

Cette astuce permet de profiter de la pression dite "gratuite" (la pression disponible étant comprise typiquement entre 11 et 20 bars) pour actionner les vérins sans utiliser d'énergie annexe (qui serait électrique ou d'air comprimé)

Pour remplacer l'air présent dans le sas de traitement par le gaz anesthésiant il convient d'ouvrir une vanne de purge (circuit D).

Pour des raisons de sécurité, on préfère mettre en place une disposition où l'injection de gaz n'est possible que si le sas est rempli par les caisses composant le lot, ainsi aucune personne ne peut être présente dans le sas pendant l'injection.

Prenons dans ce qui suit, l'exemple du cas d'une volaille de chair :
- sortie des cages précédentes et introduction d'un lot (2 mn sans injection) ;
- puis 1 mn pour monter à 20% ;
- puis 2 mn pour atteindre 35%
- puis 2 mn pour atteindre approximativement 75%
- 1mn 30 s de stabilisation à cette teneur voisine de 75%
- une minute pour redescendre à 0% de CO₂.

La présente invention concerne alors un procédé pour anesthésier des animaux avant abattage, par l'action anesthésiante de gaz ou mélanges gazeux, procédé fonctionnant en mode « lot » où les animaux vivants, préférentiellement regroupés au sein d'une ou plusieurs cages, sont positionnés dans un sas de traitement où les animaux sont mis en contact avec du gaz ou mélange gazeux d'anesthésie, en suivant un cycle comportant plusieurs étapes caractérisées par des teneurs différentes en gaz d'anesthésie, se caractérisant en ce que au moins l'une des étapes est réalisée à l'aide de gaz récupéré dans le sas et stocké dans au moins une capacité de stockage provisoire à volume variable, ladite au moins une capacité à volume variable étant apte à générer une dépression permettant d'aspirer du gaz contenu dans le sas, et à stocker le gaz ainsi récupéré de façon provisoire en vue d'une utilisation d'anesthésie ultérieure.

Selon un des modes de réalisation de l'invention, la récupération du gaz contenu dans le sas est réalisée par un système d'aspiration de type soufflet (ou accordéon), système de soufflet qui fait le caractère variable du volume de la capacité, où l'on injecte du gaz anesthésiant dans un boudin (vérin) associé à la capacité considérée, faisant se lever le soufflet, générant ainsi une dépression qui va permettre d'aspirer du gaz présent dans le sas, le gaz utilisé pour actionner le boudin étant préférentiellement libéré ultérieurement dans le sas pour être ainsi valorisé (non perdu).

Selon un des modes de réalisation de l'invention, on dispose de plusieurs capacités de stockage, permettant de stocker différentes teneurs de gaz anesthésiant dans un gaz vecteur, par exemple de l'air, allant d'une teneur la plus faible à une teneur la plus élevée.

## Revendications

1. Procédé pour anesthésier des animaux avant abattage, par l'action anesthésiante de gaz ou mélanges gazeux (4), procédé fonctionnant en mode « lot » où les animaux vivants, préférentiellement regroupés au sein d'une ou plusieurs cages, sont positionnés dans un sas (1) de traitement où les animaux sont mis en contact avec du gaz ou mélange gazeux d'anesthésie, en suivant un cycle comportant plusieurs étapes **caractérisées par** des teneurs différentes en gaz d'anesthésie, **se caractérisant en ce que** au moins l'une des étapes est réalisée à l'aide de gaz récupéré dans le sas et stocké dans au moins une capacité ((2), No1, No2, No3....) de stockage provisoire possédant un volume variable, ladite au moins une capacité à volume variable étant apte à générer une dépression permettant d'aspirer du gaz contenu dans le sas (1), et à stocker le gaz ainsi récupéré de façon provisoire en vue d'une utilisation d'anesthésie ultérieure.

2. Procédé selon la revendication 1, **se caractérisant en ce que** :
- ladite au moins une capacité destinée à recevoir le gaz récupéré est de volume variable par le fait qu'elle est munie d'un système de soufflet apte à se soulever; et
- la récupération du gaz contenu dans le sas est réalisée à l'aide de ce système de soufflet par le fait que :
i) la capacité considérée est munie d'un boudin (3);
j) on injecte du gaz anesthésiant dans le boudin (3) associé à la capacité considérée, faisant se lever le soufflet, générant ainsi une dépression qui va permettre d'aspirer du gaz présent dans le sas, le gaz utilisé pour actionner le boudin étant préférentiellement libéré ultérieurement dans le sas pour être ainsi valorisé.

3. Procédé selon la revendication 1 ou 2, **se caractérisant en ce que** l'on dispose de plusieurs capacités de stockage provisoire possédant un volume variable, permettant de stocker différentes teneurs de gaz anesthésiant dans un gaz vecteur.

4. Installation pour anesthésier des animaux avant abattage, par l'action anesthésiante de gaz ou mélanges gazeux (4), fonctionnant en mode « lot », comportant un sas (1) de traitement où les animaux sont mis en contact avec du gaz ou mélange gazeux d'anesthésie, **se caractérisant en ce qu'**elle comporte au moins une capacité ((2), No1, No2, No3....) de stockage provisoire de gaz possédant un volume variable, capacité apte à récupérer du gaz dans le sas et à le stocker de façon provisoire, ladite au moins une capacité à volume variable étant apte à générer une dépression permettant d'aspirer du gaz contenu dans le sas (1), et à stocker le gaz ainsi récupéré de façon provisoire en vue d'une utilisation d'anesthésie ultérieure.

5. Installation selon la revendication 4, **se caractérisant en ce que** :
- ladite au moins une capacité destinée à recevoir le gaz récupéré est de volume variable par le fait qu'elle est munie d'un système de soufflet apte à se soulever; et
- la capacité considérée est munie d'un boudin (3) ; et
- la récupération du gaz contenu dans le sas est réalisable à l'aide de ce système de soufflet par le fait que l'injection de gaz anesthésiant dans le boudin (3) associé à la capacité considérée, permet de faire se lever le soufflet, générant ainsi une dépression permettant d'aspirer du gaz présent dans le sas.

6. Installation selon la revendication 4 ou 5, **se caractérisant en ce qu'**elle comporte plusieurs capacités de stockage provisoire possédant un volume variable, permettant de stocker différentes teneurs de gaz anesthésiant dans un gaz vecteur.

## Patentansprüche

1. Verfahren zur Betäubung von Tieren vor der Schlachtung durch die Betäubungswirkung von Gas oder Gasgemischen (4), wobei das Verfahren im "Batch"-Modus, bei dem die lebenden Tiere, die vorzugsweise innerhalb eines oder mehrerer Käfige gruppiert sind, in einer Behandlungsschleuse (1) positioniert werden, wo die Tiere mit Betäubungsgas oder Betäubungsgasgemisch in Kontakt gebracht werden, gemäß einem Zyklus läuft, der mehrere Schritte aufweist, die durch unterschiedliche Betäubungsgasgehalte **gekennzeichnet sind, dadurch** gekennzeichnet, dass wenigstens einer der Schritte mithilfe von Gas durchgeführt wird, das in der Schleuse rückgewonnen und in wenigstens einer Kapazität ((2), No1, No2, No3....) zur provisorischen Speicherung mit variablem Volumen gespeichert wird, wobei die wenigstens eine Kapazität mit variablem Volumen geeignet ist, einen Unterdruck zu erzeugen, mit dem Gas angesaugt werden kann, das in der Schleuse (1) enthalten ist, und das so rückgewonnene Gas im Hinblick auf eine spätere Verwendung zur Betäubung provisorisch zu speichern.

2. Verfahren nach Anspruch 1, **dadurch gekennzeichnet, dass**:
- die wenigstens eine Kapazität zur Aufnahme des rückgewonnenen Gases dadurch ein variables Volumen aufweist, dass sie mit einem Faltenbalgsystem versehen ist, das geeignet ist, sich anzuheben; und
- die Rückgewinnung des Gases, das in der Schleuse enthalten ist, mithilfe dieses Faltenbalgsystems dadurch erfolgt, dass:
i) die jeweilige Kapazität mit einem Balg (3) versehen ist;
j) Betäubungsgas in den Balg (3) eingeblasen wird, der der jeweiligen Kapazität zugeordnet ist, wodurch bewirkt wird, dass sich der Faltenbalg anhebt, wodurch ein Unterdruck erzeugt wird, der es ermöglicht, Gas anzusaugen, das in der Schleuse vorhanden ist, wobei das Gas, das dafür genutzt wird, den Balg zu betätigen, vorzugsweise später in der Schleuse freigesetzt wird, um so wiederverwertet zu werden.

3. Verfahren nach Anspruch 1 oder 2, **dadurch gekennzeichnet, dass** mehrere Kapazitäten zur provisorischen Speicherung mit variablem Volumen zur Verfügung stehen, mit denen verschiedene Betäubungsgasgehalte in einem Trägergas gespeichert werden können.

4. Anlage zur Betäubung von Tieren vor der Schlachtung durch die Betäubungswirkung von Gas oder Gasgemischen (4), die im "Batch"-Modus läuft, aufweisend eine Behandlungsschleuse (1), wo die Tiere mit Betäubungsgas oder Betäubungsgasgemisch in Kontakt gebracht werden, **dadurch gekennzeichnet, dass** sie wenigstens eine Kapazität ((2), No1, No2, No3....) zur provisorischen Speicherung von Gas mit variablem Volumen aufweist, wobei die Kapazität geeignet ist, Gas in der Schleuse rückzugewinnen und es provisorisch zu speichern, wobei die wenigstens eine Kapazität mit variablem Volumen geeignet ist, einen Unterdruck zu erzeugen, mit dem Gas angesaugt werden kann, das in der Schleuse (1) enthalten ist, und das so rückgewonnene Gas im Hinblick auf eine spätere Verwendung zur Betäubung provisorisch zu speichern.

5. Anlage nach Anspruch 4, **dadurch gekennzeichnet, dass**:
- die wenigstens eine Kapazität zur Aufnahme des rückgewonnenen Gases dadurch ein variables Volumen aufweist, dass sie mit einem Faltenbalgsystem versehen ist, das geeignet ist, sich anzuheben; und
- die jeweilige Kapazität mit einem Balg (3) versehen ist; und
- die Rückgewinnung des Gases, das in der Schleuse enthalten ist, mithilfe dieses Faltenbalgsystems dadurch durchführbar ist, dass durch das Einblasen von Betäubungsgas in den Balg (3), der der jeweiligen Kapazität zugeordnet ist, bewirkt wird, dass sich der Faltenbalg anhebt, wodurch ein Unterdruck erzeugt wird, mit dem Gas angesaugt werden kann, das in der Schleuse vorhanden ist.

6. Anlage nach Anspruch 4 oder 5, **dadurch gekennzeichnet, dass** sie mehrere Kapazitäten zur provisorischen Speicherung mit variablem Volumen aufweist, mit denen verschiedene Betäubungsgasgehalte in einem Trägergas gespeichert werden können.

## Claims

1. Method for anaesthetizing animals prior to slaughter, through the anaesthetizing action of a gas or gas mixture (4), the method operating in "batch" mode in which living animals, preferably grouped together in one or more cages, are positioned in a processing airlock (1) where the animals are brought into contact with the anaesthetizing gas or gas mixture, according to a cycle comprising several steps which are **characterized by** different anaesthetic-gas contents, **characterized in that** at least one of the steps is carried out using gas recovered from the airlock and stored in at least one temporary storage capacity ((2), No1, No2, No3, etc.) of variable volume, said at least one variable-volume capacity being able to generate a reduced pressure enabling it to draw out the gas contained in the airlock (1) and store the gas thus recovered temporarily so that it can be used in a subsequent anaesthetizing operation.

2. Method according to Claim 1, **characterized in that**:
- said at least one capacity intended to receive the recovered gas is of variable volume through the fact that it is equipped with a bellows system able to rise; and
- the recovery of the gas contained in the airlock is performed using this bellows system through the fact that:
i) the capacity concerned is equipped with a tube (3);
j) anaesthetizing gas is injected into the tube (3) associated with the capacity concerned, causing the bellows to rise, thus generating a reduced pressure that will allow the gas present in the airlock to be drawn out, the gas used for actuating the tube preferably being released later into the airlock in order thus to be put to use.

3. Method according to Claim 1 or 2, **characterized in that** there are several temporary storage capacities of variable volume, enabling the storage of different contents of anaesthetizing gas in a carrier gas.

4. Installation for anaesthetizing animals prior to slaughter through the anaesthetizing action of gas or gas mixtures (4), operating in "batch" mode comprising a processing airlock (1) where the animals are brought into contact with anaesthetizing gas or gas mixture, **characterized in that** it comprises at least one temporary gas storage capacity ((2), No1, No2, No3, etc.) of variable volume, which capacity is able to recover gas from the airlock and temporarily store it, said at least one variable-volume capacity being able to generate a reduced pressure enabling it to draw out the gas contained in the airlock (1) and store the gas thus recovered temporarily with a view to use in a later anaesthetizing operation.

5. Installation according to Claim 4, **characterized in that**:
- said at least one capacity intended to receive the recovered gas is variable in volume through the fact that it is equipped with a bellows system able to rise; and
- the capacity concerned is equipped with a tube (3); and
- the gas contained in the airlock can be recovered using this bellows system through the fact that the injection of anaesthetizing gas into the tube (3) associated with the capacity concerned allows the bellows to rise, thus generating a reduced pressure enabling it to draw out the gas present in the airlock.

6. Installation according to Claim 4 or 5, **characterized in that** it comprises several temporary storage capacities of variable volume, enabling the storage of different contents of anaesthetizing gas in a carrier gas.
